# FASCICULE DE BREVET EUROPEEN

(11) **EP 0 558 360 B1**
(45) Date de publication et mention de la délivrance du brevet: **13.11.1996**
(21) Numéro de dépôt: 93400226.2
(22) Date de dépôt: 29.01.1993
(51) Int. Cl.: C02F 3/34, D21H 21/36, C12R 1/44

(54) **Procédé de traitement de la flore contaminant les circuits papetièrs mettant en oeuvre des bactéries**
Verfahren zur bakteriellen Behandlung von mit Flora verschmutzten Kreisläufen der Papierindustrie
Process for bacterial treatment of circuits in paper industry contaminated with flora

(30) Priorité: 24.02.1992 FR 9202080
(43) Date de publication de la demande: 01.09.1993
(73) Titulaire: RHONE-POULENC CHIMIE, 92408 Courbevoie Cédex (FR)
(72) Inventeur: Guerineau, Pierre, F-86580 Biard (FR); Rosli, Peter, CH-5243 Mullingen (CH)
(74) Mandataire: Ricalens, François

(56) Documents cités:
- EP-A- 0 372 520
- P.H.A. SNEATH ET AL. 'Bergey's Manual of Systematic Batceriology-Vol.2' 1987 , WILLIAMS &WILKINS , LONDON

## Description

La présente invention se rapporte à un procédé pour réguler la croissance de la flore microbienne et/ou bactérienne, levures, moisissures et entérobactéries, se développant dans les circuits d'eau en industrie papetière.

Les papiers et cartons usagés, désignés sous le terme fibres cellulosiques de récupération, constituent aujourd'hui, au même titre que le bois, une matière première importante de l'industrie papetière. Leur recyclage est généralement effectué dans les usines papetières selon le procédé suivant:

Les papiers et cartons récupérés et débarrassés de tout corps étranger du type agrafes ou plastiques par exemple, sont introduits dans un pulpeur ou désintégrateur pour y être dispersés dans de l'eau. La pâte résultante, désagrégée puis dépastillée, subit ensuite une étape de raffinage qui consiste à gonfler la fibre cellulosique par hydratation. La fabrication proprement dite avec formation d'une feuille de papier débute ultérieurement sur toile filtrante avec élimination d'eau, séchage et enfin bobinage.

La chaîne de fabrication de papier implique donc une alimentation continue en eau, cette dernière étant en partie recyclée au travers généralement de plusieurs circuits de circulation d'eau annexes.

Le recyclage partiel de cette eau dans la chaîne de fabrication est bien entendu propice au développement d'une flore bactérienne. Cette flore est indésirable car elle provoque la formation de slimes.

Par slimes, on désigne en industrie papetière les dépôts, chimiques ou biologiques, observés dans le circuit de fabrication. Il peut s'agir de fibres, de carbonates ou autres charges minérales ou de bactéries. En se décrochant des cuviers ou tuyaux où ils se sont accumulés, ces slimes provoquent des casses du papier et taches colorées. Il en résulte des arrêts fréquents de la chaîne de fabrication et une détérioration des conditions de travail.

Actuellement, les solutions proposées pour limiter la formation des slimes mettent en oeuvre des agents désinfectants chimiques ou encore biocides. Il s'agit généralement de produits chimiques organochlorés et organobromés qui inhibent efficacement le développement de la flore contaminante.

Toutefois, en raison de leur origine chimique, ces composés ne donnent pas entière satisfaction.

Ils sont très toxiques et non biodégradables.

Leur relargage même partiel dans l'environnement soulève d'importants problèmes écologiques et plus particulièrement pose un problème d' épuration des eaux contaminées.

Enfin, pour obtenir une inhibition efficace de la flore bactérienne, il est nécessaire d'utiliser plusieurs biocides en alternance ainsi que des doses de plus en plus élevées. Il est clair que l'introduction répétée de ces doses importantes accentue ce problème de pollution.

En conséquence, l'application des biocides au traitement de la flore bactérienne est aujourd'hui très critiquée pour des motifs écologiques et on souhaite s'en affranchir.

La présente invention a précisément pour objectif de proposer un substitut aux biocides, qui soit doté d'une efficacité comparable mais qui en revanche ne perturbe pas l'environnement.

Dans la demande EPO372520A2 déposée au nom du docteur Oberkofler, il est décrit un procédé permettant de réduire ou d'éviter l'utilisation de biocide dans les circuits d'usine à papier, en introduisant des microorganismes ou plutôt un mélange de micro-organismes.

Plus précisément, la présente invention se rapporte à un procédé utile pour réguler la croissance de la flore microbienne et/ou bactérienne se développant dans les circuits d'eau en industrie papetière caractérisé en ce qu'il met en oeuvre à titre d'agent désinfectant principal une quantité efficace de bactéries du genre Staphylocoques ou Staphylococcus Carnosus.

Par agent désinfectant on entend selon l'invention un composé capable de contrôler et plus précisément de stopper le développement de la flore biologique indésirable se développant dans les circuits papetiers.

La flore microbienne et/ou bactérienne désigne de manière générale la flore contaminant les circuits d'eau en industrie papetière. Elle est généralement constituée de levures, moisissures et d'entérobactéries.

A titre de microorganismes représentatifs de cette flore, on peut en particulier citer les moisissures du genre Penicillium, Mucor ou Aspergillus, les levures comme les Candida et Rhodotorula, les bactéries aérobies du genre Pseudomonas, Enterobacter ou Bacillus et les Clostridiums à titre de bactéries anaérobies.

La diversité de ces microorganismes nécessite classiquement la mise en oeuvre simultanée de plusieurs biocides.

De manière inattendue, il a été mis en évidence que les bactéries sélectionnées selon l'invention inhibent efficacement le développement de l'ensemble de cette flore contaminante. Elles sont actives vis à vis de l'ensemble des microorganismes précités et ne nécessitent pas de manière générale la mise en oeuvre d'un quelconque biocide supplémentaire. Aucune formation de slimes n'est observée en leur présence et la fabrication du papier est assurée sans incident quelconque.

Les bactéries employées selon l'invention sont plus particulièrement des bactéries du genre Staphylocoques et de l'espèce Staphylococcus Carnosus et plus particulièrement de celle commercialisée sous la dénomination M 72 par la société TEXEL et dont les caractéristiques sont présentées dans l'exemple ci-après, d'un de ses mutants ou de ses recombinants.

Les bactéries en particulier celles de l'espèce staphylocoques carnosus sont non corrosives et dotées d'une totale innocuité. La bactérie M 72 est en particulier déjà employée en industrie alimentaire. En conséquence, leur relargage partiel dans l'environnement ne soulève aucun problème écologique.

Il s'agit en outre de préférence de souches thermosensibles c'est-à-dire qui sont détruites par simple chauffage. Cette caractéristique offre un avantage supplémentaire par rapport aux biocides. En fin de chaîne de fabrication, les bactéries résiduelles demeurant dans la feuille de papier humide seront détruites lors du séchage de cette feuille avant l'étape de bobinage. En ce qui concerne les biocides, ceux- ci peuvent demeurer à l'état de traces dans le papier final.

Les bactéries selon l'invention peuvent être introduites à n'importe quel point du circuit papetier. Toutefois, pour des raisons de commodité, elles sont de préférence injectées en tête de chaîne de fabrication.

Leur dosage dans le circuit papetier est bien entendu fonction du pH, de la température, du volume d'eau en circulation et de la quantité de papier traitée dans ce circuit. Généralement, il se situe entre 1.10¹⁰ et 10.10¹³ bactéries par tonne de papier à traiter. Selon un mode préféré de l'invention, la quantité en bactéries est comprise entre 1.10¹² et 10.10¹² bactéries par tonne de papier.

Un ensemencement quotidien des eaux utilisées avec des bactéries selon l'invention s'avère suffisant pour inhiber efficacement le développement de la flore bactérienne. Toutefois, cet ensemencement peut être bien entendu renouvellé une ou deux fois.

Les bactéries utilisées selon l'invention sont en outre sans effet sur les performances des autres additifs classiquement mis en oeuvre dans les circuits papetiers, de type dispersants, antimousses ou agents de rétention par exemple.

Les bactéries peuvent éventuellement être combinées à d'autres agents désinfectants sous réserve bien entendu que ces derniers soient inoffensifs à leur égard.

L'analyse de prélèvements d'eau d'une chaîne de fabrication de papier traitée selon le procédé de l'invention confirme le non développement de la flore habituelle.

L'efficacité surprenante des bactéries selon l'invention à l'égard de cette flore bactérienne peut vraisemblablement s'expliquer de la manière suivante :

Les microorganismes présents dans les circuits d'eau trouvent des conditions de croissance optimales grâce à des conditions de pH, de température et d'oxygènation favorables et à un excédent d'offre de substances nutritives organiques et inorganiques présent en permanence dans le système.

Avec les biocides classiques on déplace l'équilibre biologique du système en réduisant le taux de bactéries. On n'empêche pas le développement de nouvelles bactéries.

Dans le cas du procédé selon l'invention, c'est le développement des bactéries qui est perturbé. Les bactéries selon l'invention privent les bactéries contaminantes de leurs éléments nutritifs. il n'y a plus développement de nouvelles bactéries contaminantes ce qui a pour effet immmédiat de stopper complètement la formation de slimes.

Les bactéries selon l'invention produisent en outre de la nitratoréductase. Cette enzyme réduit les nitrates en nitrites qui possèdent eux un effet bactériostatique. Les eaux en industrie papetière étant très riches en nitrates, il est vraisemblable que cette activité bactériostatique complète l'activité privative précitée desdites bactéries.

Enfin, toute bactérie peut produire des bactéricides qui contribueront également à l'élimination d'une partie de la flore contaminante.

Outre son efficacité désinfectante et sa compatibilité avec l'environnement, le procédé selon l'invention offre des avantages supplémentaires d'ordre économiques et techniques.

L'emploi des bactéries selon l'invention permet de stopper efficacement les casses, trous, taches et autres problèmes de production causés habituellement par les slimes. Il s'en suit une rentabilité accrue du procédé de fabrication.

Enfin, l'amélioration de la qualité bactériologique des eaux, la réduction de leur toxicité, la suppression des mauvaises odeurs et le meilleur fonctionnement de la machine à papier, observés avec le procédé selon l'invention, contribuent également à l'amélioration des conditions de travail du personnel des industries papetières.

L'exemple présenté ci-après permettra de mettre en évidence d'autres avantages et caractéristiques de l'invention sans toutefois en limiter la portée.

### EXEMPLE

Les bactéries utilisés à titre d'agent désinfectant sont des bactéries commercialisées par la société TEXEL sous la dénomination M72.

Il s'agit plus précisément de Staphylococcus Carnosus possédant les caractéristiques suivantes:

### Caractéristiques morphologiques :

Coques se présentant seuls, par paires, en tétrades ou amas irréguliers.

### Caractéristiques physiologiques:

- gram positif,
- asporogène,
- non mobile,
- catalase positive,
- homofermentaire(pas de production de dioxyde de carbone),
- température de croissance: 12 - 42°C,
- température optimale de croissance: 25 - 32°C.

### Caractéristiques biochimiques:

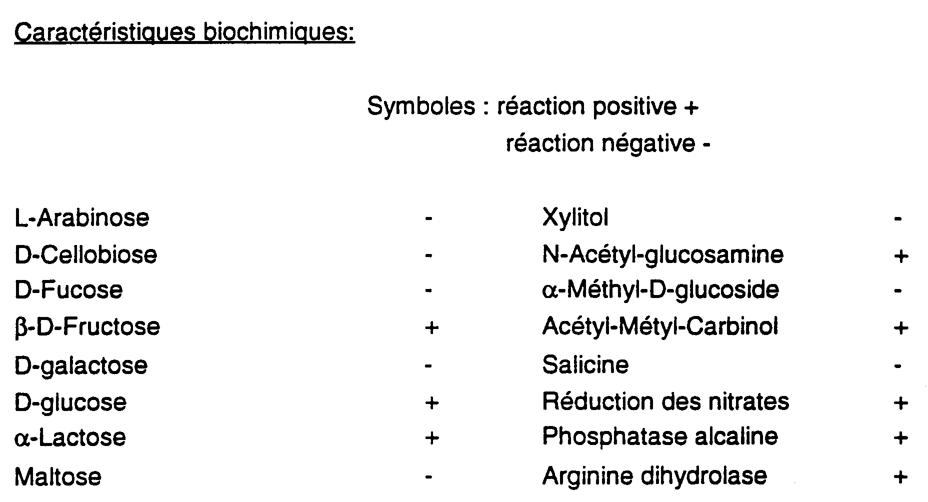

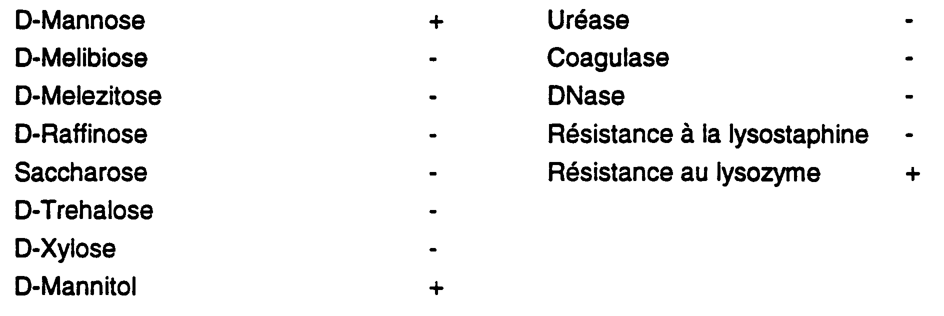

L'activité de ces bactéries a été testée dans une industrie de papeterie sur un circuit de fabrication classique.

Les bactéries sont introduites une fois par jour dans le pulpeur ou cuvier machine à un taux d'ensemencement de l'ordre de 5.10¹² bactéries par tonne de papier traitée. Dans le cas présent la production de papier s'évalue entre environ 25 à 30 tonnes par jour. On effectue quotidiennement des prélèvements d'eau en plusieurs points du circuit principal et secondaire et leur concentration en moisissures, bactéries et levures est déterminée par des méthodes de dosages classiques sur des milieux de cultures adéquats.

### Milieu de recherche des coliformes

- bouillon lactosé à la bile et au vert brillant,
- gélose lactosée au désoxycholate.

### Milieu de recherche des entérocoques

- gélose D-coccosel.

### Milieu de recherche des staphylocoques pathogènes

- gélose Barid-Parker au Tellurite.

### Milieu de recherche des anaérobies sulfito-réducteur-clostridium

- gélose V-F -(viande-foie).

### Milieu de recherche des moisissures/levures contaminantes

- gélose G-M (extrait de malt)

### Milieu de recherche de la flore totale mésophile aérobie

- gélose PCA enrichie en lait.

Le tableau ci-après rend compte des résultats.

Dans ce tableau, à T égal à zéro, figure la concentration d'origine des eaux en flore microbienne. Les bactéries selon l'invention sont introduites quotidiennement à partir de T égal à 1.

On note très rapidement une chute de la concentration de la flore microbienne vers une valeur proche de zéro. Ces mesures témoignent de l'efficacité du procédé selon l'invention. On note, à partir de T égal à 28, qui correspond à l'arrêt de l'approvisionnement des eaux en bactéries, une recrudescence très rapide de la flore microbienne.

**TABLEAU**

| | CONCENTRATION (x 1000) | | |
|---|---|---|---|
| T (jours) | Entérobactéries | Moisissures | Levure |
| •To | 1180 | 860 | 1020 |
| 1 j. début du traitement | | | |
| 2 j. | 950 | 580 | 780 |
| 3 j. | n.d. | 300 | n.d. |
| 4 j. | 580 | 100 | 220 |
| 5 j. | 150 | <70 | 160 |
| 6 j. | 100 | 50 | 100 |
| 7 j. | 50 | <20 | 50 |
| 8j. à 26 j. | <0,01 | <0,01 | <0,01 |
| 27j arrêt du traitement | | | |
| 28 j. | 380 | 200 | 580 |
| 29 j. | 740 | 420 | 780 |
| 30 j. | 1100 | 500 | 700 |
| * n.d. = non déterminé. | | | |

## Revendications

1. Procédé utile pour réguler la croissance de la flore microbienne et/ou bactérienne se développant dans les circuits d'eau en industrie papetière caractérisé en ce qu'il met en oeuvre à titre d'agent désinfectant principal une quantité efficace de bactéries de l'espèce Staphylococcus Carnosus.

2. Procédé selon la revendication 1 caractérisé en ce qu'il s'agit d'une bactérie produisant un nitratoréductase.

3. Procédé selon l'une des revendications précédentes, caractérisé en ce que les bactéries sont introduites à un taux variant entre 1.10¹⁰. à 10.10¹³ bactéries par tonne de papier à traiter.

4. Procédé selon l'une quelconque des revendications 1 à 3 caractérisé en ce que les bactéries sont introduites à un taux compris de préférence entre 1. 10¹² et 10.10¹² bactéries par tonne de papier.

5. Procédé selon l'une quelconque des revendications 1 à 4 caractérisé en ce que l'on introduit au moins une fois par jour lesdites bactéries dans les circuits d'eau.

6. Procédé selon l'une quelconque des revendications précédentes caractérisé en ce qu'il peut mettre en outre en oeuvre un ou plusieurs autre(s) agent(s) désinfectant(s) compatibles avec lesdites bactéries.

7. Procédé selon l'une quelconque des revendications précédentes caractérisé en ce que ladite bactérie présente la taxonomie suivante :
Caractéristiques morphologiques :
Coques se présentant seuls, par paires, en tétrades ou amas irréguliers.
Caractéristiques physiologiques:
- gram positif,
- asporogène,
- non mobile,
- catalase positive,
- homofermentaire(pas de production de dioxyde de carbone),
- température de croissance: 12 - 42°C,
- température optimale de croissance: 25 - 32°C.
Caractéristiques biochimiques:

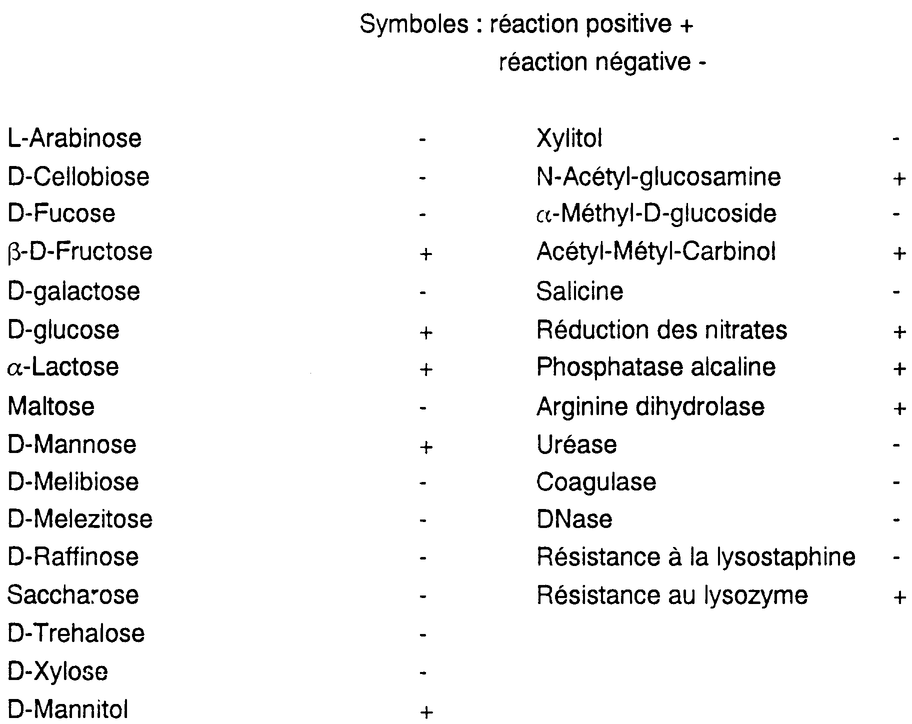

## Claims

1. Process which is useful for regulating the growth of the microbial and/or bacterial flora that grows in the water circuits in the papermaking industry, characterised in that it employs as main disinfectant agent an effective amount of bacteria of the species Staphylococcus carnosus.

2. Process according to Claim 1, characterised in that the bacterium in question is one which produces a nitrate reductase.

3. Process according to one of the preceding claims, characterised in that the bacteria are introduced at a level varying between 1×10¹⁰ and 10×10¹³ bacteria per tonne of paper to be treated.

4. Process according to any one of Claims 1 to 3, characterised in that the bacteria are introduced at a level preferably between 1×10¹² and 10×10¹² bacteria per tonne of paper.

5. Process according to any one of Claims 1 to 4, characterised in that the said bacteria are introduced at least once a day into the water circuits.

6. Process according to any one of the preceding claims, characterised in that it can, in addition, employ one or more other disinfectant agent(s) which are compatible with the said bacteria.

7. Process according to any one of the preceding claims, characterised in that the said bacterium possesses the following taxonomy:
Morphological characteristics:
Cocci occurring alone, in pairs, in tetrads or irregular clumps.
Physiological characteristics:
- Gram-positive,
- asporogenous,
- non-mobile,
- catalase-positive,
- homofermentative (no carbon dioxide production),
- growth temperature: 12 - 42°C,
- optimal growth temperature: 25 - 32°C.
Biochemical characteristics:

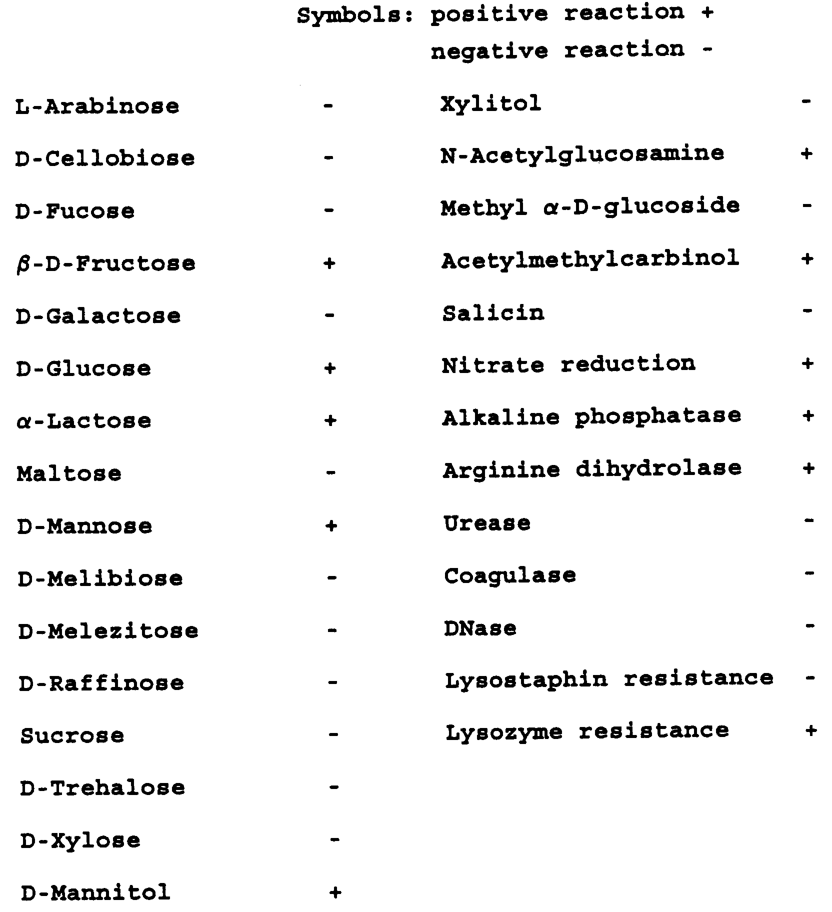

## Patentansprüche

1. Verfahren, das zur Kontrolle des Wachstums von mikrobieller und/oder bakterieller Flora, die sich in Wasserkreisläufen der Papierindustrie entwickelt, nützlich ist, dadurch gekennzeichnet, daß dabei als hauptsächliches Desinfektionsmittel eine wirksame Menge von Bakterien der Gattung Staphylococcus Carnosus eingesetzt wird.

2. Verfahren nach Anspruch 1, dadurch gekennzeichnet, daß es sich um ein Bakterium handelt, das eine Nitratreduktase erzeugt.

3. Verfahren nach einem der vorhergehenden Ansprüche, dadurch gekennzeichnet, daß die Bakterien in einer Menge eingeführt werden, welche von 1 x 10¹⁰ bis 10 x 10¹³ Bakterien pro Tonne zu behandelndem Papier variiert.

4. Verfahren nach jedem der Ansprüche 1 bis 3, dadurch gekennzeichnet, daß die Bakterien in einer Menge eingeführt werden, welche vorzugsweise zwischen 1 x 10¹² und 10 x 10¹² Bakterien pro Tonne Papier enthalten ist.

5. Verfahren nach einem der Ansprüche 1 bis 4, dadurch gekennzeichnet, daß man wenigstens ein Mal pro Tag die Bakterien in den Wasserkreislauf einführt.

6. Verfahren nach einem der vorhergehenden Ansprüche, dadurch gekennzeichnet, daß man des weiteren ein oder mehrere weitere(s) Desinfektionsmittel, das bzw. die mit den Bakterien kompatibel ist bzw. sind, einsetzen kann.

7. Verfahren nach einem der vorhergehenden Ansprüche, dadurch gekennzeichnet, daß die Bakterien die folgende Taxinomie zeigen:
Morphologische Eigenschaften:
Kokken zeigen sich einzeln, paarweise, als Tetraden oder unregelmäßige Anhäufungen.
Physiologische Eigenschaften:
- grammpositiv,
- asporogen,
- nicht beweglich,
- Katalase positiv,
- homofermentativ (keine Produktion von Kohlendioxid),
- Wachstumstemperatur: 12 - 42°C,
- optimale Wachstumstemperatur: 25 - 32°C.
Biochemische Eigenschaften:
